# EUROPÄISCHE PATENTSCHRIFT

(11) **EP 2 624 914 B1**
(45) Veröffentlichungstag und Bekanntmachung des Hinweises auf die Patenterteilung: **16.07.2014**
(21) Anmeldenummer: 11773181.0
(22) Anmeldetag: 04.10.2011
(51) Int. Cl.: A61N 5/10

(54) **VORRICHTUNG ZUR BEWEGUNG EINES OBJEKTS**
DEVICE FOR MOVING AN OBJECT
DISPOSITIF DE DÉPLACEMENT D'UN OBJET

(30) Priorität: 05.10.2010 DE 102010047356
(43) Veröffentlichungstag der Anmeldung: 14.08.2013
(73) Patentinhaber: Rheinisch-Westfälische Technische Hochschule Aachen (RWTH), 52062 Aachen (DE)
(72) Erfinder: ARENBECK, Henry, 47198 Duisburg (DE); ABEL, Dirk, 52072 Aachen (DE); EBLE, Michael, 52074 Aachen (DE)
(74) Vertreter: Cohausz Hannig Borkowski Wißgott
(86) Internationale Anmeldenummer: PCT/EP2011/004916
(87) Internationale Veröffentlichungsnummer: WO 2012/045428

(56) Entgegenhaltungen:
- MONICA SERBAN ET AL.: "A deformable phantom for 4D radiotherapy verification: Design and image registration evaluation", MED. PHYS., Bd. 35, Nr. 3, 28. Februar 2008 (2008-02-28), Seiten 1094-1102, XP002665381,

## Beschreibung

Die Erfindung betrifft eine Vorrichtung zur Bewegung eines Objekts umfassend eine Trennwand, die eine in der Trennwand sphärisch gelagerte Linear-Durchführung aufweist, durch die ein Schubelement linear verschieblich hindurchgeführt ist, wobei das Schubelement an seinem einen Ende einen Träger zur Befestigung des Objektes aufweist und der Träger durch Bewegung des anderen Endes des Schubelementes im Raum positionierbar ist.

Die Erfindung betrifft weiterhin auch ein robotisches Phantom zur Nachbildung der Bewegung von Gewebeelementen gemäß eines physiologisch realistischen Musters sowie ein Verfahren zum Testen / zur Validierung einer klinischen Apparatur, insbesondere einer diagnostischen, bevorzugt radiologischen, oder einer therapierenden, insbesondere Strahlung anwendenden Apparatur.

Solche eingangs genannten Vorrichtungen sind im Stand der Technik bekannt und werden z.B. eingesetzt, wenn ein Objekt innerhalb einer abzutrennenden Umgebung von einem von dieser Umgebung getrennten Benutzer gehandhabt werden soll. Eine Anwendung ergibt sich z.B. bei der Handhabung von radioaktivem Material, von dem eine Person durch die genannte Trennwand getrennt ist und dieses Material durch die Trennwand hindurch mittels des Schubelementes beliebig im Raum positionieren kann, z.B. mittels eines Greifers, der als Träger des radioaktiven Objektes an einem Ende des Schubelementes befestigt ist. Die Positionierbarkeit ergibt sich zum Einen durch Bewegung des Objektes mit dem Schubelement um den Mittelpunkt des sphärischen Gelenkes der Durchführung und zum Anderen durch lineare Verschiebung des Schubelementes in der Lineardurchführung, d.h. Abstandsänderung zum Mittelpunkt des Gelenks.

Allgemein erschließt eine solche Vorrichtung durch die Trennwand die Möglichkeit der Ortsmanipulation eines Objektes in einer anderen Umgebung als die, in der die manipulierende Person sich befindet.

Dabei wird es als nachteilig empfunden, dass bislang solche Vorrichtungen nur zur manuellen Bedienung am Markt erhältlich sind. Ortsmanipulationen, die eine reproduzierbare Bewegung / Positionierung eines Objektes erfordern, können daher mit solchen bekannten Vorrichtungen nicht oder zumindest nicht mit einer genügenden Genauigkeit ausgeführt werden.

Weiterhin ist es bekannt, dass im Laufe der letzten Jahre sich der Fokus in der medizinischen Bildgebung und der Dosisapplikation in der Strahlentherapie verschoben hat von statischen (3D) zu dynamischen Strukturen (4D).

Techniken zur Erfassung von Gewebe oder Schwerpunktbewegungen sowie bewegungssynchrone Dosisapplikation werden aktuell entwickelt und verfeinert. Als Konsequenz gewinnen Mittel zur adäquaten Validierung solcher Techniken mehr und mehr an Bedeutung.

Sowohl in der Bildgebung als auch in der Strahlentherapie wird die Validierung solcher Mittel traditionell auf Basis von sogenannten Phantomen durchgeführt, die die relevanten Aspekte der physikalischen Umgebung im Körper eines zu untersuchenden oder zu behandelnden Lebewesens, z.B. eines Menschen nachbilden.

Sollen 4D-Technologien beurteilt werden, so beinhalten die nachzubildenden Aspekte die Bewegung von Gewebeelementen nach einem physiologisch realistischen Muster. 4D-Phantome, die bisher entwickelt wurden, verfügen nicht über die Fähigkeit, die Trajektorie eines Objektes im Inneren der nachgebildeten anatomischen Umgebung, z.B. eines Organs, beliebig einzustellen und mit einfachen Mitteln sensorisch zu erfassen. Darüber hinaus kann der klinische Anwendungsumfang im Allgemeinen nicht verändert werden.

Es ist daher die Aufgabe der Erfindung, eine Vorrichtung der eingangs genannten Art weiterzubilden und mit einer solchen Vorrichtung eine genaue und reproduzierbare Positionierung / Bewegung von grundsätzlich beliebigen Objekten zu ermöglichen.

In einer Anwendung, welche die Erfindung jedoch nicht beschränkt, soll weiterhin eine neue Art von Phantom vorgeschlagen werden, das die zuvor genannten Nachteile nicht aufweist.

Anwendungsunabhängig soll eine Vorrichtung bereit gestellt werden, mit der auf einfache Weise beliebige messbare translatorische Bewegungen erzeugt werden können. Es ist weiterhin die Aufgabe, eine modulare mechanische Struktur bereit zu stellen, die bevorzugt einen kosteneffizienten, einfachen oder sogar "fliegenden" Austausch bewegter Objekte oder eine Erweiterung von klinischen Komponenten ermöglicht.

Das erfindungsgemäße mit der Vorrichtung gebildete Phantom soll sowohl in der Bildgebung als auch in der Strahlentherapie Verwendung finden können.

Gemäß der Erfindung wird die Aufgabe dadurch gelöst, dass bei einer Vorrichtung der eingangs genannten Art an dem anderen Ende des Schubelementes um das Ende herum wenigstens drei Gelenke befestigt sind, von denen wenigstens zwei als sphärische Gelenke ausgebildet sind, wobei jedes dieser Gelenke über einen Antrieb mit einem an der Trennwand befestigten Gelenk verbunden ist, wobei von den an der Trennwand befestigten Gelenken wenigstens zwei als sphärische Gelenke ausgebildet sind und mit jedem Antrieb der Abstand zwischen zwei verbundenen Gelenken einstellbar ist.

Dabei wird hier im Rahmen der Erfindung unter einem sphärischen Gelenk allgemein jedes Gelenk verstanden bei dem zwei gelenkig verbundene Gelenkteile um einen gemeinsamen Mittelpunkt herum bewegbar sind.

Der wesentliche Kerngedanke der Erfindung beruht darauf, statt einer manuellen Manipulation eines Objektes an dem einen Ende des Schubelementes, das sich auf der einen Seite der Trennwand befindet, eine automatisierte Bewegung des Objektes zu erzielen, wobei von der Umgebung des Objektes durch die Trennwand getrennt die dafür vorgesehenen Antriebe auf der anderen Trennwandseite angeordnet sind. So besteht die Möglichkeit ein Objekt automatisiert in einer Umgebung zu positionieren oder zu bewegen, in welcher die zur Automatisierung vorgesehenen Antriebe nicht untergebracht werden können. Hierfür können die Antriebe z.B. durch eine übergeordnete Steuereinheit jeweils individuell angesteuert werden, um bei jedem der Antriebe den gewünschten Abstand zwischen den verbundnen Gelenken einzustellen.

Jedes der hier genannten sphärischen Gelenke weist dabei zwei Gelenkteile auf, die um einen Mittelpunkt herum sphärisch zueinander bewegbar sind. So ist ein jedes der sphärischen Gelenke, die an dem Ende des Schubelementes angeordnet sind, das dem Träger gegenüberliegt, mit einem seiner Gelenkteile an diesem Ende befestigt und mit dem anderen Gelenkteil am Antrieb, bzw. einem von den Elementen, aus denen sich der Antrieb zusammensetzt.

Ähnliches gilt für die sphärischen Gelenke an der Trennwand. Dort ist ein Gelenkteil mit der Trennwand z.B. fest, alternativ auch um die Normale zur Trennwand rotierbar befestigt und das andere Gelenkteil mit dem Antrieb verbunden bzw. mit einem von den Elementen, aus denen sich der Antrieb zusammensetzt.

Sofern ein sphärisches Gelenk als kardanisches Gelenk ausgebildet ist, weist es drei Gelenkteile auf, von denen ein mittleres zwei sich schneidende Drehachsen hat und die zwei äußeren Gelenkteile diejenigen bilden, die um den Mittelpunkt bewegbar sind.

Durch die Anlenkung an wenigstens drei Orten an der Trennwand und drei korrespondierenden Orten am Ende des Schubelementes mit den dazwischen liegenden längenverstellbaren Antrieben kann dieses Ende und damit auch der gegenüberliegende Träger des Schubelementes sowohl um den Mittelpunkt des sphärischen Gelenkes der Lineardurchführung verkippt werden als auch in seinem Abstand zu diesem Mittelpunkt verstellt werden. Innerhalb eines erschlossenen Bewegungsbereiches lassen sich so beliebige Ortspositionen im Raum für ein am Träger befestigtes Objekt einstellen.

In einer Ausführung kann es vorgesehen sein, dass von den wenigstens drei Gelenken am Ende des Schubelementes eines als ein nicht-sphärisches Gelenk ausgebildet ist, insbesondere bei welchem somit die beiden Gelenkteile nicht um einen Mittelpunkt, sondern nur um eine Drehachse beweglich sind.

Z.B. kann diese Drehachse in einer möglichen Ausführung radial zur Längserstreckung des Schubelementes liegen. In einer anderen Ausführung kann diese Drehachse in einem radialen Abstand senkrecht zur Längserstreckungsrichtung des Schubelementes liegen. Z.B. kann in dieser Ausführung die Gabel eines Kardangelenks drehfest mit dem Schubelement verbunden sein. Bei diesen Ausführungen können alle Gelenke an der Trennwand als sphärische Gelenke ausgebildet sein.

Bei diesen Ausführungen besteht auch die Möglichkeit, das Gelenk an der Trägerwand, welches über den Antrieb mit dem nicht-sphärischen Gelenk des Endes des Schubelementes verbunden ist, ebenfalls nicht-sphärisch auszubilden, insbesondere wobei die Drehachsen beider nicht-sphärischer Gelenke desselben Antriebs parallel liegen.

Durch eine der vorgenannten Ausführungen wird der gelenkigen Aufhängung des Schubelementes wenigstens ein Freiheitsgrad genommen, insbesondere so dass dieses um dessen Längsachse drehfest ist.

In einer anderen Ausführung besteht auch die Möglichkeit, alle Gelenke am Ende des Schubelementes sphärisch auszubilden und von den Gelenken an der Trennwand eines als nicht-sphärisches Gelenk auszubilden mit derselben Wirkung.

In wieder anderer Ausführung können alle Gelenke am Ende des Schubelementes und der Trennwand als sphärische Gelenke ausgebildet sein, Eine Drehhemmung des Schubelementes um dessen Längsachse kann sodann durch andere Mittel erfolgen, wie später beschrieben wird.

In einer bevorzugten Ausführung kann es vorgesehen sein, dass jeder Antrieb identisch aufgebaut ist und einen Schrittmotor, eine Gewindespindel und eine dazu passende Mutter aufweist, wobei der Schrittmotor an einem von zwei einander zugeordneten sphärischen Gelenken und die Mutter an dem anderen befestigt ist. Durch Drehung der Spindel mittels des Schrittmotors wird so die am Gelenk nicht rotierbar befestigte Mutter längs der Spindelachse auf der Spindel verschoben und so der Abstand zwischen Mutter und Schrittmotor und somit gleichzeitig der Abstand zwischen den Gelenkmittelpunkten verändert.

Als besonders vorteilhaft wird es dabei angesehen, wenn der Schrittmotor über ein sphärisches Gelenk an der Trennwand befestigt ist und die Mutter über ein sphärisches Gelenk am Ende des Schubelementes. Hierfür kann am Ende des Schubelementes eine Befestigungsbasis vorgesehen sein, an der die jeweils dieser Basis zugewandten Gelenkteile der wenigstens drei sphärischen Gelenke befestigt sind.

Sofern die drei am Ende des Schubelementes angebrachten Gelenke z.B. als kardanische Gelenke ausgebildet sind ist an einem jeweils bezogen auf das Schubelement äußeren Gelenkteil die jeweilige Spindelmutter befestigt und die Befestigungsbasis bildet ein gemeinsames inneres Gelenkteil der wenigstens drei Gelenke. Zwischen innerem gemeinsamen und den äußeren Gelenkteilen hat jedes der Gelenke ein mittleres Gelenkteil mit zwei sich schneidenden Achsen, die im Schnittpunkt den Gelenkmittelpunkt definieren.

Diese mittleren Gelenkteile können dabei jeweils um eine Achse senkrecht zur Längserstreckung des Schubelementes rotierbar an der Befestigungsbasis befestigt sein. Um eine wiederum dazu jeweils senkrechte Achse ist die Spindelmutter zusammen mit dem äußeren am mittleren der Gelenkteile befestigt.

Bei den sphärischen Gelenken der Trennwand können bei kardanischer Ausführung die an der Trennwand befestigten Gelenkteile jeweils eine Drehachse aufweisen, die tangential liegt zu einem um die Lineardurchführung in einer Ebene parallel zur Trennwand liegenden Kreis. Die jeweiligen am Aktor befestigten Gelenkteile können eine dazu senkrechte Achse aufweisen und die jeweils mittleren Gelenkteile weisen beide Achsen auf, die sich im Gelenkmittelpunkt schneiden.

Alternative Ausführungen der sphärischen Gelenke von Schubelement, Trennwand und Durchführung können auch von aussen gefasste Kugelkörper aufweisen, die in der Fassung um den Kugelmittelpunkt drehbar sind.

In weiterhin bevorzugter Ausführung können die Gelenke an der Trennwand, sowie die Gelenke am Ende des Schubelementes in derselben Winkelteilungum die Längsachse des Schubelementes herum angeordnet sein, insbesondere wobei die Gelenkmittelpunkte zweier Gelenke auf der Mittelachse der jeweiligen verbindenden Gewindespindel liegen. Bei kardanischen Gelenken an der Trennwand und am Ende des Schubelementes ist es weiterhin vorteilhaft, wenn in einer Grundposition, in welcher das Schubelement senkrecht zur Trennwand ausgerichtet ist, eine Achse eines Trennwandgelenks parallel ist zu einer Achse eines Schubelement-Gelenks.

In einer bevorzugten Ausführung kann am Träger des Schubelementes ein Objekt lösbar befestigt sein. Dazu kann der Träger zum Beispiel als Flansch ausgebildet sein und ein zu befestigendes Objekt weist einen entsprechenden Gegenflansch auf. Auch kann eine Bajonettverbindung zwischen Objekt und Träger realisiert sein.

Um zu gewährleisten, dass bei einer Positionierung oder Bewegung keine Rotation um die Längsachse des Schubelementes vorkommt, kann es in einer bevorzugten Weiterbildung vorgesehen sein, dass das Schubelement bezüglich seiner Längsachse drehfest durch die Linear-Durchführung hindurchgeführt ist. Z.B. kann der Querschnitt senkrecht zur Längsachse von der Kreisform abweichend ausgebildet sein. In einem solchen Fall ist der freie Querschnitt der Durchführung entsprechend angepasst.

Da jedoch gerade Durchführungen für Stangen mit rundem Querschnitt besonders preisgünstig und präzise gefertigt werden können ist es bevorzugt vorgesehen, dass das Schubelement aus wenigstens zwei, bevorzugt wenigstens drei Schubstangen mit Kreisquerschnitt gebildet ist, welche die Mittellängsachse des so gebildeten gesamten Schubelementes insbesondere in gleichmäßiger Winkelteilung umgeben, wobei jede Schubstange durch eine Führungshülse der Linear-Durchführung hindurchgeführt ist, insbesondere wobei jede Führungshülse in derselben kardanisch in der Trennwand gelagerten Gelenkplatte angeordnet ist. Bei dieser Ausführung ist ebenso eine Rotierbarkeit um die Mittellängsachse vermieden. Die Lineardurchführung ist hier durch wenigstens drei Führungshülsen gebildet.

Besonders - aber nicht ausschließlich - für die eingangs genannten klinischen Anwendungen kann es vorgesehen sein, dass die Trennwand eine Wand eines flüssigkeitsgefüllten/flüssigkeitsfüllbaren Behältnisses bildet, so dass ein am Träger befestigtes Objekt innerhalb der Flüssigkeit positionierbar / bewegbar ist. Z.B. kann das Behältnis mit Wasser gefüllt sein, um für ein mit dem Objekt simulierten Organ eine menschliche Umgebung simulieren zu können.

Es kann so mit der beschriebenen Vorrichtung in einer Anwendung ein robotisches Phantom zur Nachbildung der Bewegung von Gewebeelementen gemäß eines physiologisch realistischen Musters gebildet werden, wenn das am Träger befestigte Objekt ein Gewebeelement simuliert, und wobei weiterhin eine Steuereinheit vorgesehen ist, die eingerichtet ist, durch Ansteuerung der wenigstens drei Antriebe eine physiologisch realistische Bewegung des Objektes durchzuführen. Es kann so z.B. eine Atembewegung des Organs simuliert werden und unter den so simulierten Bedingungen eine Diagnose- oder Behandlungsapparatur getestet werden.

Weiterhin besteht die Möglichkeit, anstatt Wasser ein elastisches Material zu verwenden, das die umgebende Lunge nachbildet. Ebenso können auch feststehende Attrappen, z.B. zur Nachbildung der Rippen oder allgemein von Knochen, in das Behältnis eingefügt werden. Vorteilhafter Weise erschließt sich so die Möglichkeit zur Annäherung an die reale Physiologie eines Lebenwesens, z.B. des Menschen.

Mit der Vorrichtung können somit jegliche diagnostische oder auch therapeutische Apparaturen vor ihrer eigentlichen Anwendung an einem Lebewesen, z.B. am Menschen, an einem Phantom getestet und validiert werden, das eine physiologisch realistische Bewegung eines Organ in dessen Organumgebung nachbildet.

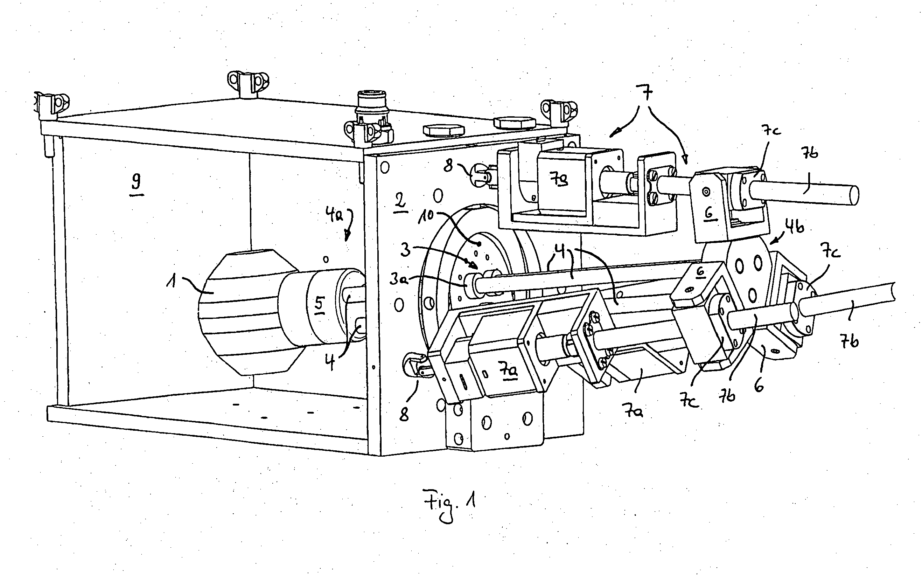

Ein Ausführungsbeispiel der Erfindung ist in der nachfolgenden Figur 1 beschrieben.

Die Figur beschreibt die Vorrichtung in der Anwendung als robotisches Phantom für klinische Anwendungen. Ohne Beschränkung auf diese weiterhin beschriebene Anwendung kann die Vorrichtung in jeder Anwendung eingesetzt werden, bei der ein wie auch immer geartetes Objekt in einem begrenzten Raum dreidimensional positioniert oder auch bewegt werden soll.

In der Figur 1 ist die Vorrichtung unterteilt durch die Trennwand 2 in ein mechanisches Modul, in der Figur 1 rechts von der Trennwand 2, das die Bewegung eines beliebigen Objekts 1 generiert, und das klinische Modul, links von der Trennwand 2, das die menschliche Physiologie nachbildet und in dieser Anwendung z.B. einer bildgebenden oder Strahlen anwendenden Apparatur zum Test / zur Validierung dieser Apparatur ausgesetzt wird.

Der modulare Aufbau wird aus zwei Gründen verwendet: Zum Einen minimiert die räumliche Trennung der Antriebskomponenten 7 deren ungewünschte Beeinflussung klinischer Eigenschaften des Phantoms. Zum Anderen ermöglicht eine leichte Austauschbarkeit von klinischen Elementen, insbesondere Objekten 1 eine hohe Vielfalt von klinischen Anwendungen.

Im Folgenden werden alle wichtigen Eigenschaften des robotischen Phantoms detaillierter beschrieben.

Das Objekt 1 ist starr verbunden mit seinem Träger 5, der sich frei innerhalb des klinischen Moduls bewegen kann und am in dieses Modul durch die Trennwand 2 hineinragenden Ende 4a des Schubelementes 4 angeordnet ist, das hier aus drei Schubstangen besteht.

Die Positionierung des Trägers 5 und somit des Objektes 1 erfolgt vollständig im mechanischen Modul. Dort schränkt die Führung des Schubelementes 4, bestehend aus einer Gelenkplatte 10 eines Kardangelenks und einer Lineardurchführung aus drei Führungshülsen 3a, die Bewegung des Schubelementes 4 bzw. des Trägers 5 in drei Freiheitsgraden ein. Bei den verbleibenden Freiheitsgraden handelt es sich um die Raumwinkelpositionierung relativ zum Mittelpinkt des Kardangelenkes, das zwischen der Durchführung 3 und der Trennwand 2 ausgebildet ist und um den Abstand des Trägers 5 bzw. des Objektes 1 von diesem Mittelpunkt durch axiale Verschiebung des Schubelementes 4. In den zur Verfügung stehenden Freiheitsgraden der Bewegung wird mit der Hilfe von drei Antrieben 7 die Positionen des Objektes 1 eingestellt.

Jeder Antrieb besitzt einen identischen Aufbau umfassend eine drehbar gelagerte Gewindestange 7b, die sich in der zugehörigen Mutter 7c bewegt. Die drehbar gelagerte Gewindestange 7b ist mittelbar über einen Schrittmotor 7a über ein Kardangelenk 8 mit der Trennwand 2 verbunden. Die Mutter 7c ist über ein Kardangelenk 6 mit dem anderen Ende 4b des Schubelements 4 verbunden. Hierfür sind alle Enden der Schubstangen in einem gemeinsamen Befestigungselement befestigt, um welches herum in gleichmäßige Winkelteilung von hier 120 Grad die drei Kardangelenke 6 angeordnet sind.

Durch Einstellung der relativen Verdrehung zwischen Mutter 7c und Gewindestange 7b durch Ansteuerung des jeweiligen Schrittmotors 7a können beliebige Abstände zwischen den beiden genannten Kardangelenken 6 und 8 realisiert werden (unter Berücksichtigung der passiv erzeugten und aus der Kinematik resultierenden Verdrehung).

Durch Zusammenspiel aller drei Antriebe 7 können der freie Endpunkt 4b des Schubelementes 4 außerhalb des klinischen Moduls und mit ihm das Objekt 1 (auf der anderen Seite der Trennwand im klinischen Modul) frei im Sinne ihrer translatorischen Koordinaten positioniert werden. Die kinematische Struktur des robotischen Phantoms zeigt eine parallele Charakteristik, die geringe Trägheitskräfte und eine hohe kinematische Präzision ermöglicht. Elf bewegte Körper werden durch 63 kinematische Bindungen beschränkt.

Spezialisierte Algorithmen ermöglichen eine effiziente Koordinatentransformation: Die Vorwärtskinematik, das ist die Berechnung der translatorischen Raumposition des Targets aus gegebenen Motorpositionen, erfordert die Auswertung von nur drei impliziten nichtlinearen Beziehungen. Inverskinematik, das ist die Berechnung der Motorpositionen aus einer gegebenen translatorischen Raumposition des Targets, kann explizit durchgeführt werden. Dies wird dadurch ermöglicht, dass die Kardangelenke 6,8 der jeweiligen Antriebe 7 einen Schnittpunkt mit der Drehachse der jeweils zugehörigen Spindelstange 7b bilden.

Die Realisierung des mechanischen Moduls hat eine maximale kinematische Genauigkeit zum Ziel. Um dies zu erreichen, werden bevorzugt ausschließlich hochpräzise Gelenke 6,8 verwendet und eine hohe Steifigkeit aller mechanischen Komponenten sichergestellt. Als Konsequenz kann die Position des Objekts 1 basierend auf kinematischer Berechnung und somit indirekt mit hoher Genauigkeit erfasst werden.

Somit sind nicht nur die Erzeugung, sondern auch die Messung der Bewegung bzw. Positionen ins mechanische Modul verlagert. Die Grundmaterialien des mechanischen und des klinischen Moduls sind z.B. Aluminium bzw. Plexiglas. Das Schubelement 4, die einzige mechanischen Komponente, die in den klinischen Bereich hineinragt, kann in einer bevorzugten Ausführung aus einem Kohlefaserwerkstoff bestehen.

Das klinische Modul kann in einer möglichen Ausgestaltung einen Tank 9 oder sonstiges Behältnis 9 ausbilden, der/das zum Zwecke klinischer Experimente mit Wasser befüllt werden kann, wodurch ein menschenähnliches Dichteumfeld geschaffen wird.

Im Arbeitsraum, der sich aus den kinematischen Eigenschaften und geometrischen Dimensionen des Phantoms ergibt, findet bei einer möglichen Ausführung der Erfindung ein zumindest im Wesentlichen kugelförmiges Volumen von 70 mm Durchmesser Platz. Innerhalb dieses Volumens kann der Schwerpunkt des Objekts kollisionsfrei positioniert werden. Die Ausdehnung dieses Volumens ist ausreichend zur Reproduktion von Bewegungen, die innerhalb des menschlichen Körpers auftreten können.

In einer bevorzugten Ausbildung wird jeder Antrieb durch einen 2-Phasen Schrittmotor in Bewegung versetzt. Die Achsposition jedes Schrittmotors kann sensorisch, z.B. mit Hilfe eines 3-Kanal Inkrementalencoders überwacht werden und die Messwerte einer Steuereinheit übermittelt oder von dieser abgefragt werden. Die Koordination aller drei Motorachsen erfolgt mit Hilfe einer Steuereinheit basierend auf Bewegungsbefehlen, z.B. die von einem Host-PC übermittelt werden können. Neben der Ansteuerung der Motoren 7a liest in bevorzugter Weiterbildung die hier nicht gezeigte Steuereinheit kontinuierlich die Achspositionen aus. Achspositionen und Steuerkommandos werden z.B. über eine Ethernet-Schnittstelle übertragen.

Es kann demnach vorgesehen sein, eine übergeordnete externe Steuereinheit bereit zustellen, welche die Positionierung und/oder Bewegung des Objektes koordiniert. Die Steuereinheit liest die Achspositionen der Antriebssensoren, insbesondere der Inkrementalgeber und/oder steuert die Schrittmotoren 7a an.

Zur Positionserfassung werden die translatorischen Koordinaten des Objekts im kartesischen Raum aus den Achspositionen jedes Motors hergeleitet. Diese Herleitung kann durch die Steuereinheit oder einen daran angeschlossenen Rechner erfolgen. Dies ermöglicht eine einfache Sensortechnologie bei gleichzeitig hoher Genauigkeit und Updaterate der Sensorinformationen, erfordert aber, die Sensordaten einer Vorwärtskinematik-Transformation zu unterziehen, was rechentechnisch aufwändig ist. Um Echtzeit-Positionstracking zu ermöglichen, kann es daher vorgesehen sein, die Vorwärtskinematik-Transformation zu ersetzen durch eine Response Surface Approximation, die basierend auf einem homogenen Sampling im Arbeitsraum erzeugt wird. Da sich der Arbeitsraum durch eine kontinuierliche und glatte Transformation zwischen Objekt- und Achskoordinaten auszeichnet, kann eine hohe Genauigkeit der Approximation erreicht werden.

Insbesondere für klinische Anwendungen kann es vorgesehen sein, mehrere verschiedene Objekte austauschbar am Träger des Schubelementes 4 befestigen zu können. Hierfür können am Objekt und am Träger korrespondierende Flansche vorgesehen sein.

Zwei verschiedene Objekte sollen exemplarisch beschrieben werden, die je nach Bedarf am Träger 5 befestigt werden können. Diese Elemente sind vorgesehen für eine Verwendung in der Strahlentherapie (RT) bzw. Positronen-Emissions-Tomografie (PET).

Beide Objekte umfassen eine innere und eine äußere Kugel, die konzentrisch angeordnet sind. Das Strahlentherapie-Objekt umfasst einen Plexiglas-Vollmaterialkern im Innern, der vollständig bedeckt ist durch eine Schicht aus Holz konstanter Dicke. Der Plexiglas-Kern simuliert Weichgewebe, z.B. einen Lungentumor. Die Holzschicht repräsentiert das den Tumor umgebende Lungengewebe niedrigerer Dichte. Das Objekt ist bevorzugt in Scheiben geschnitten, deren Schnittebenen jeweils sowohl das Innen- als auch das Außenmaterial teilen. Radiochromic-Filme sind zwischen den Scheiben platziert, mit deren Hilfe die Dosisverteilung im Raum und speziell am Dichtesprung beim Übergang vom Tumor zur Lunge gemessen werden kann.

Das PET-Target ist aus hohlen Kunststoffkugeln aufgebaut. Die innere und die äußere Kugel werden unabhängig voneinander mit einem Fluid hoher bzw. niedriger Aktivität gefüllt. Durch die Füllung wird das Target sichtbar in PET mit einer realistischen räumlichen Aktivitätsverteilung. Dies ermöglicht eine Validierung von 4D-PET-basierten Messungen der Objekt-Bewegung.

## Patentansprüche

1. Vorrichtung zur Bewegung eines Objekts (1) umfassend eine Trennwand (2), die eine sphärisch gelagerte Linear-Durchführung (3) aufweist, durch die ein Schubelement (4) linear verschieblich hindurchgeführt ist, wobei das Schubelement (4) an seinem einen Ende (4a) einen Träger (5) zur Befestigung des Objektes (1) aufweist und der Träger (5) durch Bewegung des anderen Endes (4b) des Schubelementes (4) im Raum positionierbar ist, **dadurch gekennzeichnet, dass** an dem anderen Ende (4b) des Schubelementes (4) um das Ende herum wenigstens drei Gelenke (6) befestigt sind, von denen wenigstens zwei als sphärische Gelenke ausgebildet sind, wobei jedes dieser Gelenke (6) über einen Antrieb (7) mit einem an der Trennwand (2) befestigten Gelenk (8) verbunden ist, wobei von den an der Trennwand befestigten Gelenken wenigstens zwei als sphärische Gelenke ausgebildet sind und mit jedem Antrieb (7) der Abstand zwischen zwei verbundenen Gelenken (6,8) einstellbar ist.

2. Vorrichtung nach Anspruch 1, wobei jedes der Gelenke (6) am Ende des Schubelements (4) und/oder jedes der Gelenke (8) an der Trennwand (2) als sphärisches Gelenk ausgebildet ist.

3. Vorrichtung nach einem der vorherigen Ansprüche, wobei jeder Antrieb einen Schrittmotor (7a), eine durch diesen angetriebene Gewindespindel (7b) und eine dazu passende Mutter (7c) aufweist, wobei der Schrittmotor (7a) an einem der Gelenke (8) und die Mutter (7c) an einem anderen der Gelenke (6) befestigt ist.

4. Vorrichtung nach einem der vorherigen Ansprüche, wobei die Gelenke (6,8), insbesondere die sphärischen Gelenke (6,8) als kardanische Gelenke (6,8) ausgeführt sind und die Gelenke (8) an der Trennwand (2), sowie die Gelenke (6) am Ende des Schubelementes (4) in derselben Winkelteilung angeordnet sind, insbesondere wobei die Gelenkmittelpunkte auf der Mittelachse der Gewindespindel (7b) liegen.

5. Vorrichtung nach einem der vorherigen Ansprüche, wobei das Schubelement (4) bezüglich seiner Längsachse drehfest durch die Linear-Durchführung hindurchgeführt ist.

6. Vorrichtung nach Anspruch 5, wobei das Schubelement (4) wenigstens zwei, bevorzugt wenigstens drei Schubstangen mit Kreisquerschnitt aufweist und jede Schubstange durch eine Führungshülse (3a) der Linear-Durchführung (3) hindurchgeführt ist, insbesondere wobei jede Führungshülse (3a) in derselben kardanisch in der Trennwand (2) gelagerten Gelenkplatte (10), insbesondere um eine gemeinsame Mittelachse angeordnet ist.

7. Vorrichtung nach einem der vorherigen Ansprüche, wobei die Trennwand (2) eine Wand eines flüssigkeitsgefüllten/flüssigkeitsfüllbaren Behältnisses (9) bildet, insbesondere welches eine der menschlichen Physiologie nachempfundenen Umgebung bildet, so dass ein am Träger (5) befestigtes Objekt (1) innerhalb der Flüssigkeit positionierbar/bewegbar ist.

8. Robotisches Phantom zur Nachbildung der Bewegung von Gewebeelementen gemäß eines physiologisch realistischen Musters, wobei es eine Vorrichtung nach einem der vorherigen Ansprüche umfasst und das am Träger (5) befestigte Objekt (1) ein Gewebeelement simuliert, wobei eine Steuereinheit vorgesehen ist, die eingerichtet ist, durch Ansteuerung der wenigstens drei Antriebe (7) eine physiologisch realistische Bewegung des Objektes (1) durchzuführen.

9. Robotisches Phantom nach Anspruch 8, wobei das Objekt eine Kugel, insbesondere eine Hohlkugel, insbesondere eine gefüllte Hohlkugel umfasst.

10. Robotisches Phantom nach Anspruch 8 oder 9, wobei das Objekt zwei zueinander konzentrische Kugeln aufweist, die unterschiedlich dichte Materialien umfassen.

11. Robotisches Phantom nach Anspruch 9 oder 10, wobei das Objekt in mehrere parallele Schichten geteilt ist, insbesondere die wenigstens zum Teil die wenigstens eine, insbesondere beide Kugeln schneiden, wobei zwischen den Schichten radiochromischer Film anbringbar / angebracht ist.

12. Verfahren zum Testen / zur Validierung einer klinischen Apparatur, insbesondere einer diagnostischen, bevorzugt radiologischen, oder einer therapierenden, insbesondere Strahlung anwendenden Apparatur, **dadurch gekennzeichnet, dass** mit einer Vorrichtung nach einem der vorherigen Ansprüche ein durch das Objekt (1) simuliertes Gewebeelement durch Ansteuerung der wenigstens drei Antriebe (7) in eine physiologisch realistische Bewegung versetzt wird.

## Claims

1. Device for moving an object (1), comprising a partition (2) which has a spherically mounted linear feedthrough (3), through which a thrust element (4) is guided in a linearly displaceable manner, wherein the thrust element (4) has, at one end (4a) thereof, a support (5) for securing the object (1), and the support (5) can be positioned in the space by moving the other end (4b) of the thrust element (4), **characterized in that**, at the other end (4b) of the thrust element (4), at least three joints (6) are secured around said end, at least two of which joints are designed as spherical joints, wherein each of these joints (6) is connected via a drive (7) to a joint (8) secured on the partition (2), wherein at least two of the joints secured on the partition are designed as spherical joints, and the distance between two connected joints (6, 8) can be adjusted by each drive (7).

2. Device according to Claim 1, wherein each of the joints (6) at the end of the thrust element (4) and/or each of the joints (8) on the partition (2) is designed as a spherical joint.

3. Device according to one of the preceding claims, wherein each drive has a stepping motor (7a), a threaded spindle (7b) driven by the latter, and a matching nut (7c), wherein the stepping motor (7a) is secured on one of the joints (8), and the nut (7c) is secured on another of the joints (6).

4. Device according to one of the preceding claims, wherein the joints (6, 8), in particular the spherical joints (6, 8), are designed as cardan joints (6, 8), and the joints (8) on the partition (2), and also the joints (6) at the end of the thrust element (4), are arranged in the same angular division, in particular wherein the centre points of the joints lie on the central axis of the threaded spindle (7b).

5. Device according to one of the preceding claims, wherein the thrust element (4) is guided through the linear feedthrough in a rotationally fixed manner with respect to its longitudinal axis.

6. Device according to Claim 5, wherein the thrust element (4) has at least two, preferably at least three thrust rods with a circular cross section, and each thrust rod is guided through a guide sleeve (3a) of the linear feedthrough (3), in particular wherein each guide sleeve (3a) is arranged in the same joint plate (10) mounted cardanically in the partition (2), in particular about a common central axis.

7. Device according to one of the preceding claims, wherein the partition (2) forms a wall of a receptacle (9) which is filled with liquid or can be filled with liquid, and in particular which forms an environment simulating human physiology, such that an object (1) secured on the support (5) is able to be positioned/moved inside the liquid.

8. Robotic phantom for simulating the movement of tissue elements according to a physiologically realistic pattern, wherein it comprises a device according to one of the preceding claims, and the object (1) secured on the support (5) simulates a tissue element, wherein a control unit is provided which is designed, by controlling the at least three drives (7), to execute a physiologically realistic movement of the object (1).

9. Robotic phantom according to Claim 8, wherein the object comprises a ball, in particular a hollow ball, in particular a filled hollow ball.

10. Robotic phantom according to Claim 8 or 9, wherein the object has two mutually concentric balls, which comprise materials of different density.

11. Robotic phantom according to Claim 9 or 10, wherein the object is divided into a plurality of parallel layers, in particular which at least partially intersect the at least one ball, in particular both balls, wherein radiochromic film is arranged or can be arranged between the layers.

12. Method for testing/validating a clinical apparatus, in particular a diagnostic, preferably radiological apparatus, or a therapeutic apparatus, in particular one using radiation, **characterized in that**, with a device according to one of the preceding claims, a tissue element simulated by the object (1) is set into a physiologically realistic movement by controlling the at least three drives (7).

## Revendications

1. Dispositif pour déplacer un objet (1), le dispositif comprenant une paroi de séparation (2) dotée d'un passage linéaire (3) monté sphériquement et traversé linéairement par un élément de poussée (4) coulissant, l'élément de poussée (4) présentant à une extrémité (4a) un support (5) de fixation de l'objet (1) et le support (5) pouvant être disposé dans l'espace par déplacement de l'autre extrémité (4b) de l'élément de poussée (4), **caractérisé en ce que** au moins trois articulations (6) dont au moins deux, configurées comme articulations sphériques, sont fixées autour de l'autre extrémité (4b) de l'élément de poussée (4), **en ce que** chacune de ces articulations (6) est raccordée par un entraînement (7) à une articulation (8) fixée sur la paroi de séparation (2), **en ce qu'**au moins deux des articulations fixées à la paroi de séparation sont configurées comme articulations sphériques et **en ce que** la distance entre deux articulations (6, 8) reliées l'une à l'autre peut être ajustée à l'aide de chaque entraînement (7).

2. Dispositif selon la revendication 1, dans lequel chacune des articulations (6) située à l'extrémité de l'élément de poussée (4) et/ou chacune des articulations (8) fixée sur la paroi de séparation (2) est configurée comme articulation sphérique.

3. Dispositif selon l'une des revendications précédentes, dans lequel chaque entraînement présente un moteur pas à pas (7a), une tige filetée (7b) entraînée par ce dernier et un écrou (7c) adapté à cette tige filetée, le moteur pas à pas (7a) étant fixé sur l'une des articulations (8) et l'écrou (7c) à l'autre articulation (6).

4. Dispositif selon l'une des revendications précédentes, dans lequel les articulations (6, 8) et en particulier les articulations sphériques (6, 8) sont réalisées sous la forme d'articulations (6, 8) de Cardan, les articulations (8) étant disposées sur la paroi de séparation (2) et les articulations (6) à l'extrémité de l'élément de poussée selon la même division angulaire (4), les centres d'articulation étant en particulier situés sur l'axe central de la tige filetée (7b).

5. Dispositif selon l'une des revendications précédentes, dans lequel l'axe longitudinal de l'élément de poussée (4) traverse le passage linéaire en étant bloqué en rotation.

6. Dispositif selon la revendication 5, dans lequel l'élément de poussée (4) présente au moins deux et de préférence au moins trois tiges de poussée de section transversale circulaire, chaque tige de poussée traversant une douille de guidage (3a) du passage linéaire (3), chaque douille de guidage (3a) étant disposée dans la même plaque articulée (10) montée à cardan dans la paroi de séparation (2), en particulier autour d'un axe central commun.

7. Dispositif selon l'une des revendications précédentes, dans lequel la paroi de séparation (2) forme une paroi d'un récipient (9) rempli ou apte à être rempli d'un liquide, en particulier en formant un environnement sensible à la physiologie humaine, de sorte qu'un objet (1) fixé sur le support (5) puisse être déplacé et/ou positionné à l'intérieur du liquide.

8. Simulateur robotisé destiné à imiter le déplacement d'éléments de tissu selon un motif physiologiquement réaliste, comprenant un dispositif selon l'une des revendications précédentes, l'objet (1) fixé sur le support (5) simulant un élément du tissu, avec une unité de commande conçue pour exécuter un déplacement physiologiquement réaliste de l'objet (1) par commande des trois ou plusieurs entraînements (7).

9. Simulateur robotisé selon la revendication 8, dans lequel l'objet comporte une sphère, en particulier une sphère creuse et en particulier une sphère creuse remplie.

10. Simulateur robotisé selon les revendications 8 ou 9, dans lequel l'objet présente deux sphères concentriques l'une à l'autre qui comportent des matériaux de densité différente.

11. Simulateur robotisé selon les revendications 9 ou 10, dans lequel l'objet est divisé en plusieurs couches parallèles qui découpent en particulier au moins en partie la ou les et en particulier les deux sphères, un film radiochrome pouvant être placé ou étant placé entre les couches.

12. Procédé de test et/ou de validation d'un appareillage clinique, en particulier d'un appareillage de diagnostic, de préférence radiologique, ou d'un appareillage thérapeutique, utilisant en particulier un rayonnement, **caractérisé en ce qu'**un élément de tissu simulé par l'objet (1) est déplacé selon un déplacement physiologiquement réaliste par commande des trois ou plusieurs entraînements (7) à l'aide d'un dispositif selon l'une des revendications précédentes.
